# FASCICULE DE BREVET EUROPEEN

(11) **EP 0 993 428 B1**
(45) Date de publication et mention de la délivrance du brevet: **14.08.2002**
(21) Numéro de dépôt: 98933704.3
(22) Date de dépôt: 24.06.1998
(51) Int. Cl.: C07C 37/62, C07C 39/27

(54) **PROCEDE DE CHLORATION DE PHENOLS HALOGENES**
VERFAHREN ZUR CHLORINATION VON HALOGENIERTEN PHENOLEN
METHOD FOR CHLORINATING HALOGENATED PHENOLS

(30) Priorité: 24.06.1997 FR 9707874
(43) Date de publication de la demande: 19.04.2000
(73) Titulaire: RHODIA CHIMIE, 92408 Courbevoie Cédex (FR)
(72) Inventeur: DESMURS, Jean-Roger, F-69360 Saint-Symphorien d'Ozon (FR); SPINDLER, Jean-Francis, F-69003 Lyon (FR); PADILLA, Geneviève, F-69360 Solaize (FR)
(74) Mandataire: Ricalens, François
(86) Numéro de dépôt international: FR9801332
(87) Numéro de publication internationale: WO9858893

(56) Documents cités:
- FR-A- 2 561 237
- FR-A- 2 587 332
- FR-A- 2 739 376
- US-A- 4 345 097
- K.C. SRIVASTAVA: "Synthesis of fluoro-aryloxy fatty acids and their mono-mercurated derivatives" BULLETIN OF THE CHEMICAL SOCIETY OF JAPAN, vol. 37, no. 4, avril 1964, pages 583-584, XP002057038 TOKYO, JP

## Description

La présente invention a pour objet un procédé de chloration de phénols halogénés. Elle concerne plus particulièrement la chloration de phénols halogénés dans des conditions qui défavorisent la formation de dioxine.

Il est bien connu de l'homme de métier que la chloration de phénols ou de dérivés de ces derniers, risque de conduire à la formation en quantité non négligeable, notamment du point de vue de l'environnement, de dioxine. Le risque est très amplifié lorsque la molécule de phénol comporte un atome de fluor.

Quoique cette formation soit relativement faible, elle est réputée polluante en raison de la très forte toxicité des dioxines.

Le problème est d'autant plus délicat que les catalyseurs utilisés pour la chloration du noyau sont des acides de Lewis et ces derniers sont réputés catalyser la formation de dioxine. Aussi a-t-on proposé de chlorer en l'absence de catalyseur. On a également proposé (FR-A-2.587.332) d'utiliser comme catalyseur des amines non alcoylables.

Parmi les acides de Lewis les plus couramment utilisés, on peut citer le chlorure ferrique, le chlorure de zinc, voire le chlorure cuivrique.

C'est pourquoi un des buts de la présente invention est de fournir un procédé qui permette la chloration d'halogénophénols qui minimisent la formation de dioxine.

Un autre but de la présente invention est de fournir un procédé du type précédent qui permette une bonne productivité des appareillages.

Un autre but de la présente invention est de fournir un procédé qui permette une bonne sélectivité de la chloration.

Un autre but de la présente invention est de fournir un procédé du type qui précède et qui utilise un acide de Lewis qui ne favorise pas la formation de dioxine.

Ces buts, et d'autres qui apparaîtront par la suite, sont atteints au moyen d'un procédé de chloration d'un noyau aromatique caractérisé par le fait que l'on soumet le substrat aromatique à l'action d'un agent chlorant en présence d'un acide de Lewis contenant un atome de bore lié à au moins un atome d'oxygène.

Ainsi, sur la présente invention, il a pu être trouvé des conditions de chloration, notamment de phénols halogénés, qui défavorisent la formation de dioxine(s).

Il est déjà surprenant que l'utilisation d'un acide de Lewis, fut-il particulier, n'augmente pas la teneur en dioxine(s). Il est encore plus surprenant qu'il participe à l'abaissement de la teneur en dioxine.

Les opérations de chloration particulièrement visées par la présente invention sont celles qui sont exprimées par les formules suivantes : 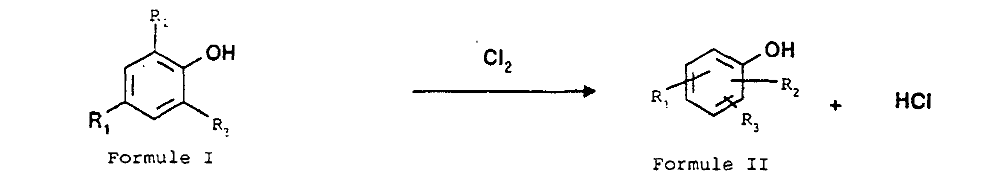 où dans la formule I, au moins un, avantageusement au moins deux des R₁, R₂, et R₃, sont hydrogènes et où au moins un des R₁, R₂, et R₃, est halogène, avantageusement fluor. Il est souhaitable que R1 soit l'halogène, avantageusement fluor. Donc il est souhaitable que l'un au moins, avantageusement les deux des R₂, et R₃, soient hydrogènes.

Dans la formule II un des hydrogènes de la formule R₁, R₂, et R₃, est remplacé par un chlore.

Parmi les chlorations les plus difficiles et les plus délicates à mener, on peut citer celle du monofluorophénol, en particulier lorsque l'atome d'halogène avantageusement de fluor est en position para par rapport au phénol. Ainsi la chloration correspondant à l'équation suivante est particulièrement à mener selon la présente invention :

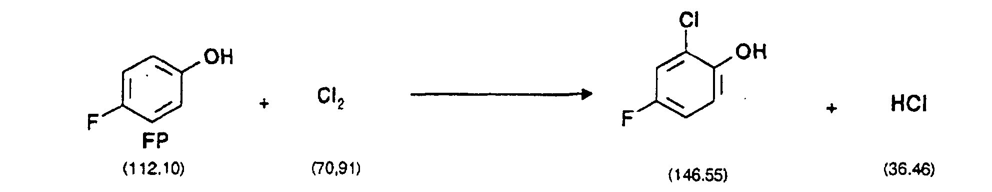

Ledit acide de Lewis contenant un atome de bore lié à au moins un atome d'oxygène peut notamment dériver de l'anhydride borique ou d'un acide borique et notamment de l'acide orthoborique.

Selon une mise en oeuvre particulièrement avantageuse de la présente invention, ledit acide de Lewis est l'anhydride borique ou un acide borique (le premier conduisant souvent au second lors de la mise en oeuvre de la chloration selon l'invention).

Mais il convient de signaler que donnent également de bons résultats les mono di, voire tri-ester, de l'acide orthoborique, avec un composé hydroxylé, alcool voire phénol. Il peut également être un acide orthoborique dans lequel un ou deux oxhydryles ont été éventuellement remplacés par un atome de fluor.

Selon la présente invention, il a été remarqué que l'utilisation de l'acide borique proprement dit (ou de son précurseur l'anhydride borique) conduisait à un effet ortho-directeur par rapport à la fonction phénol.

Le rapport entre le bore et le substrat à chlorer (exprimé respectivement en atome sur mole) est avantageusement supérieur à 1‰. de préférence à 3‰. Ce rapport est avantageusement au plus égal à environ 10% (dans la présente description le terme "environ" est employé pour mettre en exergue le fait que, lorsque le, ou les, chiffres les plus à droite d'un nombre sont des zéros, ces zéros sont des zéros de position et non des chiffres significatifs, sauf bien entendu s'il en est précisé autrement), de préférence à 5%.

Il est souhaitable que la réaction de chloration soit menée à une température comprise entre le point de fusion (commençante avantageusement finissante) du mélange réactionnel et environ 130°C.

Il convient de signaler qu'il est préférable de travailler à une température suffisante pour que la viscosité du mélange soit faible et permette ainsi une bonne dispersion du chlore gazeux. Ceci est particulièrement intéressant lorsque selon un mode avantageux de la présente invention, on évite l'utilisation de solvant.

Pour obtenir une bonne dispersion du chlore gazeux, il est préférable de soumettre le mélange réactionnel à l'action d'un mobile, et ce dans des conditions entraînant un gradient de cisaillement élevé, avantageusement au moins égal à 0,05 s⁻¹ de préférence, avantageusement compris entre 0,2 et 15 s⁻¹.

Quoique l'on puisse utiliser d'autres agents chlorants que le chlore gazeux, c'est ce dernier qui est le plus intéressant sur le plan économique.

En cas d'utilisation de chlore comme agent chlorant, il est introduit en général à une vitesse au moins égale à 0,1 m/s mais il est préférable qu'il soit introduit dans le mélange réactionnel à une vitesse au moins égale à un m/s, avantageusement à 3m/s, de préférence à 6m/s.

Il est également souhaitable que la vitesse d'introduction du chlore gazeux soit au plus égale à 30 m/s, avantageusement à 20 m/s, de préférence à 15m/s.

Il convient également de signaler qu'il est recommandé d'introduire dans le mélange réactionnel une quantité au plus égale à 1,1 fois la quantité stoechiométrique, avantageusement au plus 1,05, de préférence 1,01.

Il convient de signaler que ce qui vient d'être décrit plus haut vise essentiellement une monochloration. Il peut aussi être indiqué que la présence d'acide borique protège les réacteurs dont la surface interne est réalisée en matériau comportant de la silice ou des silicates (par exemple les verres) ; ce qui constitue un avantage supplémentaire pour l'utilisation des acides boriques'par rapport aux autres catalyseurs selon la présente invention.

Les exemples non limitatifs suivants illustrent l'invention.

### Définitions

SI = quantité de Substrat Initial ;
SF = quantité de Substrat Final ;
PF = quantité de Produit Final de réaction considéré ;
TT = taux de transformation = (SI-SF)/SI ;
RT = rendement de transformation = PF/(SI-SF) ;
RR = rendement de réaction = RTxTT = PF/SI.

### Exemple N° 1 Chloration du fluoro-4 phénol : rôle du catalyseur

La réaction de chloration a été réalisée en l'absence de solvant (Intérêt : augmentation de la productivité et suppression des solvants organochlorés).

### - Conditions réactionnelles :

T= 60°C, addition de chlore: ~ 0.15 éq./h, vitesse de sortie du chlore au niveau du plongeur : 10 m/s (valeur industrielle RQ 101 à CLY : 16 m/s), ~ 1 éq. de chlore/FP, module d'agitation Rushton (⌀ = 0.04 m, vitesse d'agitation: 1800 t/min, réacteur de 200 ml.) développant une puissance de 83 kW/m³ et engendrant une contrainte de cisaillement de 0.6 s⁻¹.

### - Résultats :

Les courbes RR = f(TT) ont été déterminées. Les résultats ci-dessous correspondent à l'optimal du couple RR/TT:

| Catalyse H₃BO₃ en éq. | dihalobenzo -dioxines : taux en ppb | constante cinétique apparente en mole⁻¹.l.s⁻¹ | rendement en (% mol.) par rapport au fluorophénol | | | | Productivité volumique (kg/m³) |
|---|---|---|---|---|---|---|---|
| | | | TT | RR chim. | RR isolé | DCFP (RR)* | |
| sans | 400 | 1,210⁻⁹ | 99,8 | 97,1 | > 95 | 2,7 | 1250 |
| 0,0035 | ≥ 3 | 4.1 10⁻⁹ | 99.8 | 98.3 | > 97 | 1.5 | 1250 |

| | | | | | | | |
|---|---|---|---|---|---|---|---|
| * rendement en dichlorofluorophénol | | | | | | | |

Ces essais indiquent qu'en présence d'acide orthoborique la réaction est à la fois plus rapide et plus sélective. En outre, la formation de benzodihalobenzodioxines (difluoro, chlorofluoro et dichloro) est fortement réduite en présence de H₃BO₃.

### Exemple N° 2 Chloration du fluoro-4 phénol : rôle de l'agitation

### - Conditions réactionnelles :

T= 60°C, addition de chlore : ~ 0,15 éq./h, vitesse de sortie du chlore au niveau du plongeur: 10 m/s, H₃BO₃: 0,0035 éq. (acide de Lewis), ~ 1 éq. de chlore/FP. Deux essais ont été réalisés avec des puissances dissipées différentes par unité de volume :
- PGN 1958 module d'agitation Rushton (⌀ = 0,04 m, vitesse d'agitation : 1800 t/min, réacteur de 200 ml.) développant une puissance de 83 kW/m³. Cet essai correspond à une agitation très optimisée qui doit limiter la réaction consécutive de formation du dichloro-2,6 fluoro-4 phénol (DCFP) - problème de micromélange.
- PGN 1965 module d'agitation Impeller (⌀ = 0,03 m, vitesse d'agitation: 1230 t/min, réacteur de 200 ml.) développant une puissance de 1,6 kW/m³.

### - Résultats :

Les courbes RR= f(TT) ont été déterminées. Les résultats ci-dessous correspondent à l'optimal du couple RR/TT:

| Puissance dissipée (kW/m³) | rendement (en % mol par rapport au fluorophénol de départ) | | | | Productivité volumique (kg/m³) |
|---|---|---|---|---|---|
| | TT | RR chim. CFP | RR isolé CFP) | RR DCFP | |
| 83 | 99,8 | 98,3 | > 97 | 1,5 | 1250 |
| 1,6 | 99 | 95,75 | > 95 | 3,25 | 1250 |

Cette réaction présente d'excellentes performances sur le plan de la sélectivité et de la productivité (PGN 1958 : TT FP = 99,8%, RR Ch CFP = 98,3%, taux de DCFP = 1,5%).

## Revendications

1. Procédé de chloration du noyau aromatique d'un phénol halogéné, **caractérisé par le fait que** l'on soumet le substrat aromatique à l'action d'un agent chlorant en présence d'acide de Lewis contenant un atome de bore lié à au moins un atome d'oxygène.

2. Procédé selon la revendication 1, **caractérisé par le fait que** ledit acide de Lewis dérive d'un acide borique.

3. Procédé selon les revendications 1 et 2, **caractérisé par le fait que** ledit acide de Lewis est un acide borique.

4. Procédé selon les revendications 1 à 3, **caractérisé par le fait que** ledit acide de Lewis est un acide orthoborique dans lequel un ou deux oxhydryles ont été éventuellement remplacés par un atome de fluor.

5. Procédé selon les revendications 1 à 4, **caractérisé par le fait que** la réaction est menée à une température comprise entre le point de fusion (commençante avantageusement finissante) du mélange réactionnel et environ 130°C.

6. Procédé selon les revendications 1 à 5, **caractérisé par le fait que** la réaction est menée en l'absence de solvant.

7. Procédé selon les revendications 1 à 6, **caractérisé par le fait que** pendant la réaction le mélange réactionnel est soumis à l'action d'un mobile réactionnel dans des condition entraînant un gradient de cisaillement au moins égal à 0,1 s⁻¹, avantageusement compris entre 0,2 et 15 s⁻¹.

8. Procédé selon les revendications 1 à 7, **caractérisé par le fait que** l'agent chlorant est le chlore gazeux.

9. Procédé selon les revendications 1 à 8, **caractérisé par le fait que** l'agent chlorant est le chlore gazeux, lequel est introduit dans le mélange réactionnel à une vitesse au moins égale à 1m/s, avantageusement à 3m/s, de préférence 6m/s.

10. Procédé selon les revendications 1 à 9, **caractérisé par le fait que** l'agent chlorant est le chlore gazeux, lequel est introduit dans le mélange réactionnel à une vitesse au plus égale à 30m/s, avantageusement à 20m/s, de préférence 15m/s.

11. Procédé selon les revendications 1 à 10, **caractérisé par le fait que** l'agent chlorant est le chlore gazeux, lequel est introduit dans le mélange réactionnel en quantité totale au plus égale à 1,1 QS (c'est-à-dire Quantité stoechiométrique), avantageusement à 1,05, de préférence à 1,0.

## Patentansprüche

1. Verfahren zur Chlorierung des aromatischen Kerns eines halogenierten Phenols, **dadurch gekennzeichnet, dass** man das aromatische Substrat der Wirkung eines Chlorierungsmittels in Gegenwart einer Lewis-Säure, welche ein Boratom enthält, das an wenigstens ein Sauerstoffatom gebunden ist, aussetzt.

2. Verfahren nach Anspruch 1, **dadurch gekennzeichnet, dass** die Lewis-Säure von einer Borsäure abgeleitet ist.

3. Verfahren nach den Ansprüchen 1 und 2, **dadurch gekennzeichnet, dass** die Lewis-Säure eine Borsäure ist.

4. Verfahren nach den Ansprüchen 1 bis 3, **dadurch gekennzeichnet, dass** die Lewis-Säure eine Orthoborsäure ist, in welcher ein oder zwei Hydroxylreste gegebenenfalls durch ein Fluoratom ersetzt wurden.

5. Verfahren nach den Ansprüche 1 bis 4, **dadurch gekennzeichnet, dass** die Reaktion bei einer Temperatur durchgeführt wird, die zwischen dem (Anfangs-, vorzugsweise dem End-) Schmelzpunkt der Reaktionsmischung und ungefähr 130°C liegt.

6. Verfahren nach den Ansprüchen 1 bis 5, **dadurch gekennzeichnet, dass** die Reaktion in Abwesenheit eines Lösungsmittels durchgeführt wird.

7. Verfahren nach den Ansprüchen 1 bis 6, **dadurch gekennzeichnet, dass** die Reaktionsmischung während der Reaktion der Wirkung eines beweglichen Körpers in der Reaktion ausgesetzt wird, unter Bedingungen, die einen Schergradienten nach sich ziehen, welcher wenigstens 0,1 s⁻¹ entspricht und vorzugsweise zwischen 0,2 und 15 s⁻¹ liegt.

8. Verfahren nach den Ansprüchen 1 bis 7, **dadurch gekennzeichnet, dass** das Chlorierungsmittel gasförmiges Chlor ist.

9. Verfahren nach den Ansprüchen 1 bis 8, **dadurch gekennzeichnet, dass** das Chlorierungsmittel gasförmiges Chlor ist, welches in die Rektionsmischung bei einer Geschwindigkeit von wenigstens 1 m/s, vorzugsweise 3 m/s, besonders bevorzugt 6 m/s eingeführt wird.

10. Verfahren nach den Ansprüchen 1 bis 9, **dadurch gekennzeichnet, dass** das Chlorierungsmittel gasförmiges Chlor ist, welches in die Reaktionsmischung bei einer Geschwindigkeit von höchstens 30 m/s, vorzugsweise 20 m/s, besonders bevorzugt 15 m/s eingeführt wird.

11. Verfahren nach den Ansprüchen 1 bis 10, **dadurch gekennzeichnet, dass** das Chlorierungsmittel gasförmiges Chlor ist, welches in die Reaktionsmischung in einer Gesamtmenge von höchstens 1,1 QS (was stoechiometrische Menge bedeutet), vorzugsweise 1,05, besonders bevorzugt 1,0 eingeführt wird.

## Claims

1. Method for chlorinating the aromatic ring of a halogenated phenol, **characterized in that** the aromatic substrate is subjected to the action of a chlorinating agent in the presence of a Lewis acid containing a boron atom bonded to at least one oxygen atom.

2. Method according to claim 1, **characterized in that** the said Lewis acid is derived from a boric acid.

3. Method according to claims 1 and 2, **characterized in that** the said Lewis acid is a boric acid.

4. Device according to claims 1 to 3, **characterized in that** the said Lewis acid is an orthoboric acid in which one or two hydroxyls have optionally been replaced by a fluorine atom.

5. Method according to claims 1 to 4, **characterized in that** the reaction is carried out at a temperature between the melting point (initial or, advantageously, final) of the reaction mixture and about 130°C.

6. Method according to claims 1 to 5, **characterized in that** the reaction is carried out in the absence of solvent.

7. Method according to claims 1 to 6, **characterized in that** during the reaction, the reaction mixture is subjected to the action of a reaction stirrer under conditions leading to a shear gradient at least equal to 0.1 s⁻¹, advantageously between 0.2 and 15 s⁻¹.

8. Method according to claims 1 to 7, **characterized in that** the chlorinating agent is chlorine gas.

9. Method according to claims 1 to 8, **characterized in that** the chlorinating agent is chlorine gas which is introduced into the reaction mixture at a velocity at least equal to 1 m/s, advantageously equal to 3 m/s, preferably 6 m/s.

10. Method according to claims 1 to 9, **characterized in that** the chlorinating agent is chlorine gas which is introduced into the reaction mixture at a velocity at most equal to 30 m/s, advantageously equal to 20 m/s, preferably 15 m/s.

11. Method according to claims 1 to 10, **characterized in that** the chlorinating agent is chlorine gas which is introduced into the reaction mixture in a total amount at most equal to 1.1 SA (that is to say stoichiometric amount) advantageously at most equal to 1.05, preferably equal to 1.0.
